# EUROPÄISCHE PATENTANMELDUNG

(11) **EP 2 238 954 A1**
(43) Veröffentlichungstag der Anmeldung: **13.10.2010**
(21) Anmeldenummer: 10158501.6
(22) Anmeldetag: 30.03.2010
(51) Int. Cl.: A61F 13/15, D04H 1/54

(54) **Materialbahn**

(30) Priorität: 06.04.2009 DE 102009016325; 14.01.2010 DE 102010000081
(71) Anmelder: McAirlaid's Vliesstoffe GmbH & Co. KG, 37339 Berlingerode (DE)
(72) Erfinder: Schmidt, Andreas, 37115, Duderstadt (DE)
(74) Vertreter: Christophersen & Partner

(57) **Zusammenfassung**

Es wird eine Materialbahn in Form einer gebundenen Faserstoffbahn (1) aus Zellstoff, bestehend aus einer Lage Zellstofffasern (2), die bereichsweise miteinander verpresst sind, die sich dadurch auszeichnet, das ein oder mehrere antimikrobielle Substanzen (3) in der Faserstoffbahn (1) als Zuschlagstoffe enthalten sind. Die Materialbahn eignet sich unter anderem als Krankenunterlage.

## Beschreibung

Die vorliegende Erfindung betrifft eine Materialbahn in Form einer Faserstoffbahn basierend auf Zellstoff, die antibakterielle Zuschlagstoffe enthält und als Krankenunterlage eingesetzt werden kann.

Mehrlagig aufgebaute Saugmatten finden im Lebensmittelbereich und auch im Hygienebereich vielfach Anwendung. So werden sie beispielsweise in Lebensmittelverpackungen als Verpackungseinlagen verwendet, um bei Fleischprodukten austretende Flüssigkeit aufzunehmen. Absorbierende hygienische Artikel auf dem Gebiet der Hygiene und der Medizin umfassen Wickeltischauflagen, Krankenunterlagen, Kopf- und Schuhschutzauflagen und dergleichen. Diesen Artikeln ist gemeinsam, dass sie Flüssigkeit aufzunehmen und zu binden vermögen.

Auf dem Gebiet der Hygiene und der Medizin werden Krankenbetten regelmäßig mit so genannten Krankenunterlagen ausgestattet, die Flüssigkeiten einschließlich Urin und Wundsekret aufnehmen sollen. Auch wenn der Patient nicht unmittelbar mit der zwischen Laken und Matratze angeordneten Unterlage in Kontakt ist, und die regelmäßig gewechselt werden, besteht die Gefahr von Infektionen durch Mikroorganismen. Durch den steten, nur über ein Laken getrennten Kontakt mit dem Patienten, haben die Krankenunterlagen konstant Körpertemperatur, eine für das Wachstum von Mikroorganismen ideale Temperatur.

Die aus dem Stand der Technik bekannten Krankenunterlagen sind der der Regel einfache Vliese, die keine antimikrobielle Ausrüstung aufweisen.

Der vorliegenden Erfindung lag die Aufgabe zugrunde, die oben beschriebenen Nachteile zu beheben bzw. zu verringern und eine antimikrobiell ausgerüstete Saugmatte zur Verfügung zu stellen, deren Saugfähigkeit und auch deren mechanische Eigenschaften durch diese Ausrüstung nicht beeinträchtigt ist.

Gegenstand der vorliegenden Erfindung ist demgemäß eine Materialbahn in Form einer Faserstoffbahn aus Zellstoff, bestehend aus einer Lage Zellstofffasern, die bereichsweise miteinander verpresst sind, welche dadurch gekennzeichnet ist, dass ein oder mehrere antimikrobiell wirkende Substanzen als Zuschlagsstoff(e) enthalten sind.

Die erfindungsgemäße Materialbahn eignet sich hervorragend zum Einsatz als Einlage im Hygienebereich, beispielsweise als Krankenunterlage.

Die antimikrobiellen Wirkstoffe, die gemäß der vorliegenden Erfindung als Zuschlagstoffe eingesetzt werden, können aus beliebigen natürlichen oder synthetischen, anorganischen oder organischen Substanzen mit antimikrobieller Wirkung ausgewählt werden, z. B. aus der Gruppe aus feinteiligem Silber und Silbersalzen, antimikrobiell wirkenden Metalloxiden, festen anorganischen und organischen Säuren, wie Benzoesäure oder Sorbinsäure, Bioziden einschließlich Bioziden auf pflanzlicher Basis, wie 2-Brom-2-nitropropan-1,3-diol (Bronopol), oder Polymere. Die antimikrobiellen Wirkstoffe können als Feststoff oder in Form von Lösungen, Emulsionen oder Suspensionen eingesetzt werden. Besonders bevorzugt werden Silber und Silberverbindungen, die vorzugsweise als feinteilige Partikel mit einer Teilchengröße zwischen 1 und 500 nm, insbesondere zwischen 1 und 100 nm, vorliegen, beispielsweise Nanosilber, oder 2-Brom-2-nitropropan-1,3-diol, eingesetzt. Die antimikrobiell wirkenden Substanzen werden üblicherweise in einer Menge zwischen 3 und 4 g Substanz/m² Faserstoffbahn enthalten. Beispiele für geeignete handelsübliche Produkte sind Silverpure® (erhältlich bei Silver Pure), SilverClear® (erhältlich bei Cilia Solutions Inc.) und Ultra-Fresh SAB-40 (erhältlich von Thomson Research Associates).

Um eine möglichst gleichmäßige Wirkung der antimikrobiellen Wirkstoffe zu erreichen, ist es bevorzugt, dass diese Substanzen gleichmäßig in der Faserstoffbahn verteilt sind. Eine gleichmäßige Verteilung der antimikrobielle wirkenden Substanzen kann beispielsweise erreicht werden, wenn diese Substanzen bereits bei der Verarbeitung von Cellulosefasern zur Faserstoffbahn eingearbeitet werden. Beispielsweise können sie in Form einer Lösung, Emulsion oder Suspension aufgesprüht werden. Feststoffe können z. B. unter Verwendung einer Hammermühle eingearbeitet werden.

Neben den erfindungsgemäß enthaltenen antimikrobiellen Wirkstoffen kann die Materialbahn weitere Zusatzstoffe enthalten. Diese Zugsatzstoffe können ausgewählt sein aus superabsorbierenden Polymeren, beispielsweise auf der Basis von Carboxymethylcellulosederivaten oder Polymeren auf der Basis von (Meth)acrylsäure- und/oder (Meth)acrylaten, und Cyclodextrine, oder weitere Hilfs- und Füllstoffe, wie Titandioxid, Kreide oder Kaolin, bzw. pflanzliche Stoffe wie Gewürze, Duftstoffe usw. sein.

Diese weiteren Zuschlagsstoffe können in einer Menge von 0,1 bis 70 Gew.-%, vorzugsweise bis 60 Gew.-%, bezogen auf die Faserstoffbahn, enthalten sein.

Die antimikrobiell wirkenden Substanzen sowie die gegebenenfalls vorhandenen weiteren Zusatzstoffe, Füll- und Hilfsstoffe sollten in einer solchen Menge in der Materialbahn vorliegen, dass dessen strukturgebende Stabilität und Reißfestigkeit für den jeweiligen Anwendungszweck noch vorhanden ist.

In einer möglichen Ausführungsform sind die antimikrobiell wirkenden Substanzen und ggf. vorhandenen Zuschlagstoffe lagenweise in die Faserstoffbahn eingearbeitet. Eine gleichmäßige antimikrobielle Wirkung wird erreicht, wenn die Substanzen möglichst homogen verteilt sind

Durch die erfindungsgemäß enthaltenen Substanzen und Zusatzstoffe können bahnförmige Filtermaterialien bzw. Absorptionsmaterialien mit einem Gesamtflächengewicht von 20 bis 600 g/m², vorzugsweise 30 bis 500g/m², hergestellt werden.

Die erfindungsgemäß eingesetzte Faserstoffbahn kann nach einem beliebigen Verfahren zur Herstellung derartiger Bahnen hergestellt werden. Vorzugsweise wird die Faserstoffbahn nach dem als Airlaid-Verfahren bekannten Verarbeitungsverfahren hergestellt. Im Airlaid-Verfahren wird ein watteähnliches Produkt aus Zellstofffasern, nämlich Fluff-pulp, in einem Luftstrom abgelegt werden und bilden eine völlig regellose Zellstofflage, die anschließend in an sich bekannter Weise partiell verdichtet wird. In einer möglichen Ausführungsform der vorliegenden Erfindung können die antimikrobiellen Wirkstoffe während des Airlaid-Verfahrens aufgebracht werden. Dazu können die Wirkstoffe, wenn in Form von Lösungen, Suspensionen oder Emulsionen vorliegen, auf den Fluff-pulp aufgesprüht werden. Es ist auch möglich, diese Wirkstoffe bei der Herstellung des Fluff-pulp, welches in der Regel durch Bearbeitung von Zellstofffasern in einer Hammermühle erhalten wird, einzuarbeiten.

In einer bevorzugten Ausgestaltung der vorliegenden Erfindung ist die Schicht aus Zellstofffasern (Faserstoffbahn) mit über die Fläche der Materialbahn verteilten Prägebereichen versehen, in denen die Zellstofffasern stärker als in den übrigen Bereichen miteinander verpresst und hierdurch klebstoff- und/oder bindemittelfrei verdichtet/verpresst sind. Es wird ein Prägemuster mit punkt-, flächen- oder linienförmigen Prägebereichen erhalten. Die Faserschicht ist demnach so strukturiert, dass die Zellstofffasern außerhalb dieser diskreten Prägebereiche gelockert übereinander oder nur schwach aneinander haftend vorliegen, wohingegen sie in den Prägebereich eine innige Verbindung mit den jeweils benachbarten Zellstofffasern eingehen. Ein völliger Verzicht auf Klebstoffe und Bindemittel zur Bildung des Verbundes aus Zellstofffasern ermöglicht ein einfaches und vollständiges Recycling. In den Prägebereichen haften die Fasern nicht lediglich aneinander. Vielmehr wird durch die Druckbeaufschlagung erreicht, dass benachbarte Zellstofffasern in diesen Prägebereichen fest und innig miteinander verbunden sind. Durch die Prägung wird erreicht, dass die Oberfläche der Materialbahn auf mindestens einer Seite eine dreidimensionale Struktur aufweist. Das Prägemuster kann eine beliebige Form haben, beispielsweise kann es einen Schriftzug oder ein Symbol darstellen.

In einer weiteren Ausgestaltung der vorliegenden Erfindung können die Zuschlagstoffe auch auf die bereits bereichsweise verdichtete Materialbahn aufgebracht werden, z. B. durch Aufsprühen oder Bedrucken.

Bei Anwendungen, in denen die Festigkeit der Faserstoffbahnen nicht ausreichend ist, kann eine weitere Verfestigung durch zusätzliche Schmelzfasern und/oder Bindemittel oder mittels Wasservernadelung erreicht werden.

Für das Adsorptions- und/oder Absorptionsverhalten der Materialbahn kann es von Vorteil sein, wenn die Zellstofffasern der Schicht ungebleicht bzw. niedrig gebleicht oder eingefärbt sind.

In einer weiteren möglichen Ausführungsform der vorliegenden Erfindung ist die Faserstoffbahn aus einzelnen Teilmaterialbahnen aufgebaut. Die Dichte der einzelnen Teilmaterialbahnen kann unterschiedlich sein. Sie können beispielsweise durch ein Bindemittel miteinander verbunden werden. Mit dieser Ausführungsform ist es möglich, die Eigenschaften der erfindungsgemäßen Materialbahn weiter zu variieren.

Zur Erhöhung der Reißfestigkeit der Materialbahn kann diese in einer weiteren Ausgestaltung der vorliegenden Erfindung eine Armierungsbahn aufweisen, die zwischen wenigstens zwei Teilfaserstoffbahnen eingelegt und mit diesen Teilfaserstoffbahnen zu der Faserstoffbahn fest verbunden ist. Als Armierungsbahn eignet sich insbesondere eine flexible Bahn, wobei eine Netzbahn mit einer Maschenweite zwischen 15 und 50 mm² besonders bevorzugt ist. Die Armierungsbahn kann beispielsweise ein Gewebe, ein Gewirke oder ein Nonwoven - Bahn sein. Je nach Anwendungsbereich kann die Armierungsbahn auch eine durchgehende Folie mit in den Druckbereichen aufgerissenen und/oder löcherig geschmolzenen Perforationen sein. Die Auswahl des Materials für eine Armierungsbahn richtet sich in der Regel nach dem Einsatzgebiet der herzustellenden Materialbahn, beispielsweise nach den Anforderungen an die Reißfestigkeit etc.

Zur weiteren Erhöhung der Festigkeit, d.h. Reißfestigkeit und/oder Steifheit, der erfindungsgemäßen Materialbahn kann auf einer Seite der Materialbahn oder auch beidseitig eine Deckschicht aus einem flächigen Material aufgebracht werden. Bei diesem flächigen Material kann es sich insbesondere um ein textiles, vliesartiges oder folienartiges Material oder auch um ein Tissue handeln. Diese Deckschicht kann mit der Faserstoffbahn verklebt und/oder verschweißt und/oder mechanisch verbunden sein.

Die erfindungsgemäße Materialbahn wird üblicherweise in Rollenform hergestellt. Das Material ist vorzugsweise so flexibel, dass dreidimensionale Körper daraus geformt werden können. In einer möglichen Ausgestaltung der vorliegenden Erfindung wird die plissiert, d.h. in Zickzackform gefaltet.

Die erfindungsgemäße Materialbahn ist insbesondere zum Einsatz im Hygienebereich geeignet, beispielsweise als Krankenunterlage, Wickeltischauflage, Inkontinenzprodukt, Damenhygieneprodukt, Wegwerfwindel oder in einem Wundbehandlungssystem. Ein weiteres Einsatzgebiet sind Lebensmittelverpackungen, beispielsweise für Frischfleisch, Fisch, Käse usw. In diesen Bereichen werden als weitere Zuschlagstoffe Adsorptionsmittel, die insbesondere als Geruchshemmer wirken, eingesetzt. Weitere geeignete Zuschlagstoffe sind superabsorbierende Polymere, die Körperflüssigkeiten absorbieren können.

Nachfolgend wird die erfindungsgemäße Materialbahn anhand eines Ausführungsbeispieles unter Bezugnahme auf die Zeichnung näher erläutert. Darin zeigen:
- Fig. 1: einen stark vergrößerten Teilschnitt durch die Materialbahn,
- Fig. 2: eine Materialbahn in perspektivischer Ansicht von oben und
- Fig.3: einen Teilschnitt durch ein mehrlagige Materialbahn

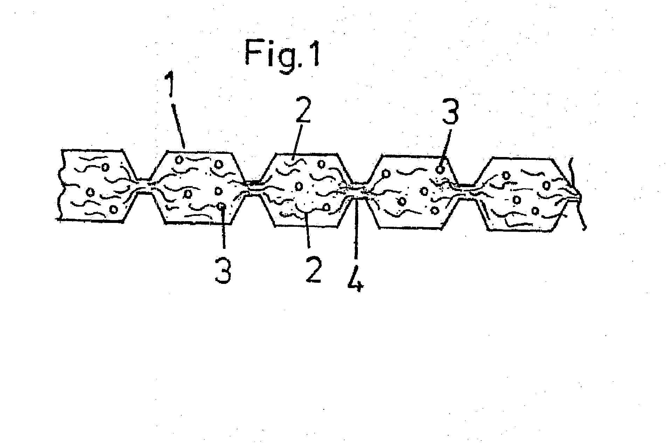

Die Fig. 1 zeigt einen Teilschnitt durch die erfindungsgemäße Materialbahn. Die Faserstoffbahn 1 besteht aus einem hohen Anteil an Zellstofffasern 2, in die Adsorptionsmittel 3 eingearbeitet ist.

Die Zellstofffasern 2 sind in dem Prägebereich 4 verdichtet und auf diese Weise miteinander verbunden. Die Prägebereiche 4 liegen sich auf der Ober- und Unterseite einander gegenüber, so dass im jeweiligen Prägebereich 4 ein nur noch schmaler Steg miteinander verbundener Zellstoffmasse verbleibt. Die übrigen, zwischen den Prägebereichen 4 angeordneten Bereiche der Schicht 1 weisen eine lockere Zellstoffschichtung auf. Eine innige Verbindung zwischen den Zellstofffasern besteht in diesen Bereichen nicht.

Die das Adsorptionsverhalten für die zu entfernenden Substanzen bestimmende Schicht 1 besteht aus Zellstofffasern 2 mit integriertem Adsorptionsmittel 3 sowie ggf. weiteren Zuschlagstoffen. Die Materialbahn 1 hat in den Prägebereichen 4 in der hier dargestellten Ausführungsform die Gestalt von Pyramidenstümpfen oder Kegelstümpfen, wobei der Winkel der hierdurch gebildeten Schrägen vorzugsweise zwischen 10° und 45° liegen sollte.

Als Zellulosematerial für die Faserstoffbahn 1 lässt sich preiswert zur Verfügung stehendes Massenmaterial einsetzen. Vorzugsweise wird ein so genannter "Fluff-pulp" eingesetzt, der sich, durch ein sehr gutes Bindungsverhalten auszeichnet, was die mechanische Festigkeit der Materialbahn gegen vertikale Zugkräfte verbessert.

Bei der Herstellung in einem kontinuierlichen Verfahren wird die später die Schicht 1 bildende Faserstoffbahn aus einer im Luftstrom aufgeschichteten Schüttung von Zellstofffasern 2 aus defiberisiertem Zellstoff (wood pulp) und antimikrobiellem(er) Zuschlagstoff(e) und ggf eingearbeiteten weiteren Zuschlagstoffen hergestellt. Für die Herstellung einer standardisierten, defiberisierten Ware kann auf die am Markt verfügbaren, nachwachsenden Holzrohstoffe zurückgegriffen werden.

Das Verfahren der Zellstoffschüttung als Ausgangsprodukt für die Schicht 1 ermöglicht eine trockene Verarbeitung der Zellstofffasern 2 und damit bei der anschließenden Prägung der mehrlagigen Bahn zwischen zwei Strukturwalzen eine sehr gute Verdichtung der Zellstofffasern in den diskreten Prägebereichen. Außerhalb der Prägebereiche 4 liegen die Fasern locker aneinander, was das Absorptionsverhalten für aufzunehmende Flüssigkeiten sowie die Flexibilität der Schicht 1 verbessert.

Die Herstellung erfolgt aus Bahnmaterial, welches in einem kontinuierlichen Prozess gefertigt wird. In luftunterstützter Schichtung werden zunächst die Zellstofffasern und die Adsorptionsmittel 3 sowie ggf. weitere Zuschlagsstoffe zur Bildung der Schicht 1 gelegt. Anschließend erfolgt in einem Kalander mit zwei strukturierten Kalanderwalzen die Herstellung der Prägebereiche 4. Ein mögliches Herstellungsverfahren wird z. B. im europäischen Patent 1 032 342 offenbart.

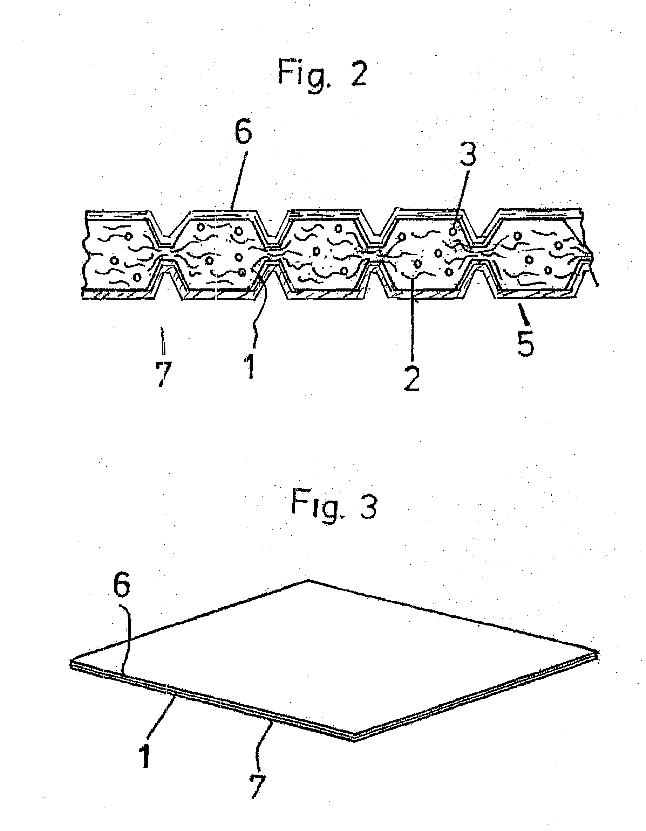

Die Zellstoffbahn 1 kann in anschließenden Verfahrensschritten zu einer mehrlagigen Bahn weiterverarbeitet werden, wie sie in Fig. 2 dargestellt ist. In diesen weiteren kontinuierlichen Schritten werden oben die Deckschicht 6 und unten die Basisschicht 7 angeordnet und mit der Kernschicht 2 verbunden. Das so hergestellte Bahnmaterial kann schließlich als Bahnmaterial verwendet und direkt kurz vor Ort für den jeweiligen Verwendungszweck oder unmittelbar im Anschluss an die Herstellung zugeschnitten oder in einen dreidimensionalen Formkörper geformt werden.

Alternativ kann die mehrlagige Bahn auch hergestellt werden, indem die Deckschicht 6 oder die Basisschicht 7 zunächst als Trägerschicht verwendet wird. Auf diese Trägerschicht 6 bzw. 7 wird die Schüttung von Zellstofffasern 2 und ggf. beigefügten antimikrobiellen Substanzen und weiteren Hilfsmitteln und Zusatzstoffen 3 im Luftstrom aufgetragen. Anschließend wird diese Anordnung aus Trägerschicht und Schüttung gemeinsam durch die Strukturwalzen des Kalanders hindurchgeführt. Alternativ kann zuvor, d.h. vor dem abschließenden Hindurchführen durch den Kalander, die andere der beiden Schichten 6, 7, welche also nicht bereits die Trägerschicht ist, noch auf die Zellstoffschicht 1 aufgelegt werden.

Die weiteren Lagen können nachträglich in an sich bekannter Weise aufgebracht werden. Werden die weiteren Lagen nachträglich aufgebracht, so können Sie eine beliebige Oberflächenstruktur aufweisen, beispielsweise glatt sein.

Die Figur 3 zeigt in einer Ansicht von schräg oben die beim Ausführungsbeispiel aus insgesamt drei Schichten oder Lagen 6, 1 und 7 zusammengesetzte Materialbahn.

Als unterste Lage dient ein Basismaterial 7 und als oberste Lage eine Deckschicht 6. Wird die Materialbahn als Krankenunterlage eingesetzt, sollte die Deckschicht 6 für die aufzunehmende Körperflüssigkeit durchlässig sein. Das Basismaterial 7 ist für diese Anwendung vorzugsweise undurchlässig, um ein durchtreten der Körperflüssigkeit(en) auf die Matratze zu verhindern. Das Basismaterial kann z. B. durch eine Folie gebildet sein.

Über dem Basismaterial 7 befindet sich, in dieser Ausführungsform vollflächig in Kontakt mit mit diesem, eine Schicht 1 aus Zellstofffasern, welche antimikrobiellen Wirkstoff(e) und ggf. noch weitere Zuschlagsstoffe enthält. Diese Schicht 1 ist für Körperflüssigkeiten durchlässig, sie werden von den Zellstofffasern der Schicht 1 aufgenommen. Bei einem Einsatz als saugfähiges Absorptionsmaterial zeigt die Schicht 1 ein hohes Absorptionsvermögen für Flüssigkeiten, insbesondere für Körperflüssigkeiten aus. Sie ist in der hier dargestellten Ausführungsform an ihrer Oberseite vollflächig mit einer Deckschicht 6 in Kontakt und durch diese abgedeckt. Die Deckschicht 6 dient zusätzlich als Schutz der Zellstoffbahn vor Schäden und Zerstörung während der Anwendung.

Um das Basismaterial 7 und die Deckschicht 6 leichter voneinander unterscheiden zu können, hat es sich als vorteilhaft erwiesen, eine oder beide Seiten zu kennzeichnen. Auf diese Weise kann z. B. Pflegepersonal auch bei hoher Arbeitsbelastung schnell erkennen, welche Seite oben und welche unten ist. Ein besonderer Vorteil, die Seiten farbig zu markieren, liegt darin, dass die Markierung durch das Bettlaken durchscheint und sichtbar ist. Beim Wechsel der Bettwäsche sieht das Pflegepersonal, dass sich unter dem Laken eine Unterlage befindet, die erst entfernt werden muss, bevor das Bettlaken in die Wäscherei gegeben wird.

Die erfindungsgemäße Materialbahn kann von weiteren Lagen umgeben sein, die größer sind als Zellstoffschicht 1 und die ggf. daran angeordneten Schichten 6 und 7, miteinander verbunden sind, ohne dass die Materialbahn miterfasst ist. Diese weiteren Lagen bilden für die Materialbahn eine Umhüllung. Sie können aus textilem, vliesartigem oder folienartigem Material oder aus Tissue sein.

### Bezugszeichenliste

- 1: Faserstoffbahn
- 2: Zellstofffasern
- 3: Zuschlagstoff
- 4: Prägebereich
- 5: mehrlagige Materialbahn
- 6: Deckschicht
- 7: Basismaterial

## Patentansprüche

**1.** Materialbahn in Form einer gebundenen Faserstoffbahn (1) aus Zellstoff, bestehend aus einer Lage Zellstofffasern (2), die bereichsweise miteinander verpresst sind,
**dadurch gekennzeichnet,**
**dass** ein oder mehrere antimikrobielle Substanzen (3) in der Faserstoffbahn (1) als Zuschlagstoffe enthalten sind.

**2.** Materialbahn nach Anspruch 1,
**dadurch gekennzeichnet**,
die antimikrobiellen Substanzen ausgewählt sind aus feinteiligem Silber und Silbersalzen, antimikrobiell wirkenden Metalloxiden, festen anorganischen und organischen Säuren, wie Benzoesäure oder Sorbinsäure, Bioziden einschließlich Bioziden auf pflanzlicher Basis.

**3.** Materialbahn nach Anspruch 2,
**dadurch gekennzeichnet**,
die antimikrobiellen Substanzen ausgewählt sind aus feinteiligem Silber und Silbersalzen, Benzoesäure oder Sorbinsäure, und/oder 2-Brom-2-nitropropan-1,3-diol.

**4.** Materialbahn nach einem der Ansprüche 1 bis 3,
**dadurch gekennzeichnet**,
die antimikrobiell wirkenden Substanzen eine Teilchengröße zwischen 1 und 100 nm aufweisen.

**5.** Materialbahn nach einem der Ansprüche 1 bis 4,
**dadurch gekennzeichnet,**
**dass** sie weitere Zuschlagstoffe ausgewählt aus superabsorbierenden Polymeren, Cyclodextrinen oder weitere Hilfs- und Füllstoffe, wie Titandioxid, Kreide oder Kaolin, enthalten sind.

**6.** Materialbahn nach einem der Ansprüche 1 bis 5,
**dadurch gekennzeichnet,**
**dass** die weiteren Zuschlagstoffe in einer Menge von 0,1 bis 70 Gew.-%, bezogen auf das Gesamtgewicht der Zellstofffasern, enthalten sind.

**7.** Materialbahn nach einem der Ansprüche 1 bis 6,
**dadurch gekennzeichnet,**
**dass** die Zuschlagstoffe (3) und/oder weiteren Zuschlagsstoffe lagenweise in die Faserstoffbahn (1) eingearbeitet sind.

**8.** Materialbahn nach einem der Ansprüche 1 bis 6,
**dadurchgekennzeichnet**,
**dass** die Zuschlagstoffe (3) und/oder weiteren Zuschlagsstoffe homogen in die Faserstoffbahn (1) eingearbeitet sind.

**9.** Materialbahn nach einem der Ansprüche 1 bis 8,
**dadurch gekennzeichnet,**
**dass** es ein Gesamtflächengewicht von 20 bis 600 g/m², insbesondere von 30 bis 500 g/m², aufweist.

**10.** Materialbahn nach einem der Ansprüche 1 bis 9,
**dadurch gekennzeichnet**,
das die Faserstoffbahn (1) nach einem Airlaid-Verfahren hergestellt wurde.

**11.** Materialbahn nach Anspruch 10,
**dadurch gekennzeichnet,**
**dass** die Zellstofffasern der Faserstoffbahn (1) in einem Prägemuster aus punkt- oder linienförmigen Prägebereichen (4) miteinander verpresst und in den Prägebereichen des Prägemusters infolge hoher Druckbeaufschlagung klebstoff- und/oder bindemittelfrei verdichtet sind.

**12.** Materialbahn nach Anspruch 11,
**dadurch gekennzeichnet,**
**dass** die Oberfläche auf mindestens einer Seite eine dreidimensionale Struktur aufweist.

**14.** Materialbahn nach einem der Ansprüche 1 bis 13,
**dadurch gekennzeichnet,**
**dass** die Bahn (1) aus einzelnen Teilfaserstoffbahnen aufgebaut ist und die Dichte der einzelnen Teilmaterialbahnen unterschiedlich sind kann.

**15.** Materialbahn nach Anspruch 14,
**dadurch gekennzeichnet,**
**dass** zwischen zwei Teilfaserstoffbahnen eine flexible Armierungsbahn eingelegt ist, die mit den Teilfaserstoffbahnen zu einer Faserstoffbahn verbunden ist.

**17.** Materialbahn nach einem der Ansprüche 1 bis 16,
**dadurch gekennzeichnet,**
**dass** auf einer Seite oder beidseitig eine Bahn (6, 7) aus textilem, vliesartigem oder folienartigem Material aufgebracht ist.

**18.** Materialbahn nach einem der Ansprüche 1 bis 17,
**dadurch gekennzeichnet,**
**dass** die Faserstoffbahn (1) mit zusätzlichen Schmelzfasern und/oder Bindemittel und oder Wasservernadelung verfestigt wurde.

**19.** Verwendung einer Materialbahn nach einem der Ansprüche 1 bis 18 als Krankenunterlage und zur Wundbehandlung.
